# EUROPÄISCHE PATENTSCHRIFT

(11) **EP 3 319 727 B1**
(45) Veröffentlichungstag und Bekanntmachung des Hinweises auf die Patenterteilung: **08.05.2019**
(21) Anmeldenummer: 16720044.3
(22) Anmeldetag: 27.04.2016
(51) Int. Cl.: B01L 3/00, C12M 1/00, C12M 1/36, G01D 3/10, G01N 33/00

(54) **VERFAHREN ZUM MESSEN EINER MEHRZAHL VON ZUSTANDSPARAMETERN EINES IN EINEM BEHÄLTER ENTHALTENEN FLUIDS**
METHOD FOR MEASURING A PLURALITY OF STATE PARAMETERS OF A FLUID CONTAINED IN A CONTAINER
PROCÉDÉ POUR MESURER UNE PLURALITÉ DE PARAMÈTRES D'ÉTAT D'UN FLUIDE CONTENU DANS UN RÉCIPIENT

(30) Priorität: 06.07.2015 DE 102015110894
(43) Veröffentlichungstag der Anmeldung: 16.05.2018
(73) Patentinhaber: Sartorius Stedim Biotech GmbH, 37079 Göttingen (DE)
(72) Erfinder: BECKER, Mario, 37085 Göttingen (DE); GRELLER, Gerhard, 37085 Göttingen (DE); GRIMM, Christian, 37308 Heilbad Heiligenstadt (DE); ADAMS, Thorsten, 37085 Göttingen (DE); BÖTTCHER, Lars, 34212 Melsungen (DE); WEICHERT, Henry, 04720 Westewitz (DE)
(74) Vertreter: Schneider, Peter Christian
(86) Internationale Anmeldenummer: PCT/EP2016/000678
(87) Internationale Veröffentlichungsnummer: WO 2017/005332

(56) Entgegenhaltungen:
- WO-A1-2013/052836
- WO-A2-2005/111602
- US-A1- 2005 272 146

## Beschreibung

### Gebiet der Erfindung

Die Erfindung bezieht sich auf ein Verfahren zum Messen einer Mehrzahl von Zustandsparametern eines in einem Behälter enthaltenen Fluids,
wobei an der Wandung des zum Einmalgebrauch konfigurierten Behälters eine Sensorträgerplatte fluiddicht festgelegt ist, welche eine Mehrzahl von Sensoren trägt, die jeweils einen mit dem Innenraum des Bioreaktors in Wirkkontakt stehenden Sensorkopf und eine mit einer externen Steuereinheit in datenaustauschender Verbindung stehende Kommunikationsstrecke aufweisen,
und wobei die externe Steuereinheit mittels der Sensorköpfe aktiver Sensoren erzeugte Messdaten über deren Kommunikationsstrecke empfängt und verarbeitet.

### Stand der Technik

Derartige Verfahren sind bekannt aus der EP 2 829 598 A2.

Diese Druckschrift offenbart einen Bioreaktor in Form eines Einweg-Bioreaktorbeutels, d.h. eines zum Einmalgebrauch als Bioreaktor konfigurierten Beutels mit flexiblen Wänden. Derartige Einweg-Bioreaktorbeutel finden zunehmend Eingang in die Produktionsprozesse der biochemischen und pharmazeutischen Industrie. Gegenüber den früher üblichen, starren und mehrfach verwendbaren Behältnissen haben die auch als Single-use-bags bezeichneten Einwegbeutel erhebliche Vorteile in Hinsicht auf Hygiene und Effizienz sowie Flexibilität von Arbeitsprozessen. Nicht unproblematisch ist dabei jedoch die Überwachung der im Beutelinneren ablaufenden Reaktionsprozesse. Dies erfolgt stets mit geeigneten Sensoren, wie bspw. elektrochemischen Sensoren, Thermo- und Leitfähigkeitssensoren, optischen und optochemischen Sensoren etc. Für jeden speziellen Reaktionsprozess im Beutelinneren müssen spezielle Sensoren zu seiner Überwachung vorgesehen sein. Aus der vorgenannten, gattungsbildenden Druckschrift ist es bekannt, die Einweg-Bioreaktorbeutel in Anlehnung an die auch früher im Zusammenhang mit starren Behältnissen verwendete Technik mit standardisierten Ports zu versehen, die eine Schnittstelle zwischen Beutelinnerem und Beuteläußerem bieten und in die bedarfsweise geeignete Sensoren eingesteckt werden. Deren Sensorkopf ragt dann in das Beutelinnere hinein, um den für den speziellen Messvorgang erforderlichen Kontakt mit dem Fluid im Beutelinneren herzustellen. Der außerhalb des Beutels angeordnete Sensorteil bietet die Schnittstelle zu einer Kommunikationsstrecke zu einer externen Steuereinheit. Diese Kommunikationsstrecke kann bspw. als elektrisches Kabel, als Glasfaserkabel oder drahtlos, bspw. in Form einer Funk- oder IR-Strecke, ausgebildet sein. Über diese Kommunikationsstrecke stehen Sensor und externe Steuereinheit in datenaustauschender Verbindung, sodass vom Sensorkopf erzeugte Daten analoger oder digitaler Natur über die Kommunikationsstrecke an die Steuereinheit versendet und dort weiterverarbeitet werden können. Die genannte Druckschrift offenbart als mechanische Schnittstelle als sogenannten Multi-Port eine stoffschlüssig in die Beutelwandung eingebrachte Platte mit einer Mehrzahl von Einzel-Durchbrüchen, in die die für den speziellen Reaktionsprozess benötigten Sensoren eingesteckt und jeweils bei Bedarf aktiviert werden. Der Schritt des Einsteckens eines Sensors in einen der Ports ist jedoch unter hygienischen Aspekten, d.h. unter Aspekten der Wahrung der Sterilität des Beutelinneren, kritisch. Hier kann die Gefahr bestehen, dass bei unsachgemäßem Vorgehen Verschmutzungen in das Beutelinnere eindringen und zu einer Kontamination des Fluids führen.

Mit der Entwicklung kostengünstiger und daher für den Einmalgebrauch geeigneter Sensoren ist es möglich geworden, die fraglichen Einwegbehälter, die als Beutel mit flexibler Wandung oder als sogenannte "Carboy"-Behälter aus Kunststoff mit starrer Wandung erhältlich sind, bereits herstellerseitig mit Einmal-Sensoren auszustatten, die unlösbar auf einer fluiddicht - z.B. durch stoffschlüssige Verbindung, etwa Verkleben oder Verschweißen, oder aber auch durch geeignete kraft- oder formschlüssige Verbindung - mit der Wandung verbundenen Sensorträgerplatte angeordnet sind. Die Konfektionierung, d.h. die Ausstattung eines individuellen Beutels mit einer individuellen Zusammenstellung unterschiedlicher Sensoren erfolgt dann unter hygienisch optimierten Bedingungen auf Seiten des Beutelherstellers und jeweils speziell nach Kundenwunsch. Entsprechende Einweg-Bioreaktorbeutel sind bspw. aus der EP 2 503 320 B1 bekannt. Dieses unter hygienischen Aspekten nicht zu beanstandende Vorgehen wirft jedoch wirtschaftliche Probleme auf, da die individuelle Konfektionierung eine kostengünstige Massenproduktion verhindert.

Aus der WO 2005/111602 A2 ist ein Überwachungssystem für Trinkwasserwerke mit einer Mehrzahl individuell aktivierbarer Sensoren bekannt. Die Aktivierung bzw. Deaktivierung der Sensoren erfolgt jeweils in Abhängigkeit von aktuellen Messwerten eben jener oder anderer Sensoren und dient der Erhöhung der Systemsicherheit durch mehrstufige Überprüfung von Messergebnissen.

### Aufgabenstellung

Es ist die Aufgabe der vorliegenden Erfindung, ein Verfahren zur Verfügung zu stellen, welches eine kostengünstige Massenproduktion von mit Einweg-Sensoren ausgestatteten Einweg-Behältern mit wenigstens bereichsweise flexibler oder wenigstens bereichsweise starrer Wandung, z.B. Einweg-Bioreaktorbeuteln oder Einweg-Mischbehältern, ermöglicht.

### Darlegung der Erfindung

Diese Aufgabe wird in Verbindung mit den Merkmalen des Oberbegriffs von Anspruch 1 dadurch gelöst, dass jedem Sensor ein Aktivierungs-Datensatz zugeordnet und für die externe Steuereinheit zugänglich hinterlegt ist und dass die externe Steuereinheit zunächst auf die Aktivierungs-Datensätze zugreift, die Sensoren dann nach vorgegebenen Regeln auf Basis ihrer jeweiligen, zugeordneten Aktivierungs-Datensätze als aktivierbare oder nicht-aktivierbare Sensoren einstuft und im Folgenden ausschließlich die als aktivierbare Sensoren eingestuften Sensoren aktiviert.

Bevorzugte Ausführungsformen sind Gegenstand der abhängigen Patentansprüche.

Dieses Verfahren ermöglicht es, sowohl den Herstellerinteressen an einer einheitlichen Massenproduktion als auch den Kundeninteressen an einer individuellen Konfektionierung und an einem an die Nutzungsmöglichkeiten gekoppelten Preis gerecht zu werden. Herstellerseitig bietet sich nämlich so die Möglichkeit, jeden Behälter mit wenigstens bereichsweise flexibler oder starrer Wandung, z.B. einen Beutel, mit einer Batterie sämtlicher im jeweiligen technischen Kontext sinnvoller Sensoren auszustatten, sodass der einzelne Behälter hinsichtlich seiner Sensorausstattung zwar aufwendiger wird, was sich jedoch durch die Kostenvorteile der einheitlichen Massenproduktion überkompensieren lässt. Andererseits erhält der Kunde jeweils Behälter, die nur über die von ihm speziell gewünschte (und bezahlte) Sensor-Funktionalität verfügen. Die individuelle Konfektionierung findet somit nicht mehr auf Seiten der Sensor-Hardware, sondern allein auf Seiten der Aktivierungs-Software, d.h. in der Ausgestaltung der Aktivierungs-Datensätze, statt.

Es bieten sich sowohl für Kunden wie für Hersteller interessante
Preisgestaltungsmöglichkeiten nach Art eines Lizenz- und Freigabemanagements an. So erhält ein erster Nutzer, der für seinen speziellen Bioreaktions- oder Mischprozess nur einen oder wenige Sensoren benötigt, zwar den gleichen Behälter, z. B. einen Beutel, mit identischer Sensorausstattung wie ein zweiter Nutzer, der für seinen Bioreaktions- oder Mischprozess eine sehr umfangreiche Sensorik benötigt. Er kauft zu seinem Behälter, z. B. seinem Beutel, jedoch lediglich eine oder wenige Sensor-Aktivierungslizenzen hinzu und zahlt einen entsprechend geringeren Preis als der zweite Nutzer, der eine Vielzahl von Sensoraktivierungslizenzen zukaufen muss.

Die Aktivierungs-Datensätze für die einzelnen Sensoren können auf unterschiedliche Weise für die externe Steuereinheit zugänglich gemacht werden. So ist es möglich, jeden Behälter mit einem individuellen Datenträger zu versehen, der dann von der externen Steuereinheit ausgelesen werden kann. Alternativ ist es auch möglich, den Nutzern die Aktivierungs-Datensätze auf anderem Wege zukommen und direkt in der externen Steuereinheit speichern zu lassen. Von besonderem Interesse ist es jedoch, die Aktivierungs-Datensätze zentral auf einem - insbesondere vom Hersteller betriebenen - Server zu hinterlegen, auf den die Nutzer über ein Netzwerk, bspw. das Internet, Zugriff haben. Unabhängig von der speziellen Art der Zurverfügungstellung der Aktivierungs-Datensätze ist es, wie der Fachmann erkennt, erforderlich, die Beutel bzw. die Sensoren individuell erkennbar zu gestalten, was jedoch auf beliebige und in unterschiedlichen Ausführungsformen bekannte Weise erfolgen kann. Rein beispielhaft sei die Verwendung von Transpondern, RFID-Chips, Barcodes, QR-Codes, etc. genannt.

Die spezielle technische Realisierung der Aktivierung einzelner Sensoren kann auf unterschiedliche Weise erfolgen, die nicht zuletzt von den jeweiligen Sensoren abhängt. So kann bei einer ersten Ausführungsform der Erfindung vorgesehen sein, dass wenigstens ein nicht aktivierter Sensor keine Messdaten erfasst. Dies kann bspw. dadurch geschehen, dass der entsprechende Sensor von der externen Steuereinheit keine zu seinem Betrieb erforderliche Versorgung, z.B. eine elektrische Energieversorgung, erhält. Im Fall optischer Sensoren kann vorgesehen sein, dass der entsprechende Sensor von einer externen Lichtquelle kein Messlicht erhält.

In vielen Fällen wird jedoch die Sensorträgerplatte mit einer zentralen Energie- und/oder Messlichtversorgung versehen sein, sodass auch nicht aktivierte Sensoren arbeitsfähig sind. Insbesondere, jedoch nicht nur in diesen Fällen, kann es günstig sein, dass wenigstens ein nicht aktivierter Sensor Messdaten erfasst und die externe Steuereinheit diese Messdaten nicht empfängt. Dies bedeutet, dass die nicht aktivierten Sensoren zwar arbeiten, die von ihnen erzeugten Messdaten aber nicht an die externe Steuereinheit übermittelt werden. Mit anderen Worten betrifft die Nicht-Aktivierung in diesem Fall also die Kommunikationsstrecke zwischen Sensor und externer Steuereinheit.

Es sind jedoch Fälle denkbar, in denen auch diese Art der Aktivierung bzw. Nicht-Aktivierung nicht funktioniert. Als Beispiel sei ein im Wesentlichen passiver optischer Sensor mit Glasfaserankopplung an die externe Steuereinheit genannt. Wird ein solcher Sensor mit Messlicht, z.B. aus einer zentralen Messlichtquelle, beschickt, wird er über seine Glasfaser-Kommunikationsstrecke zwangsläufig Ausgangslicht und damit Messdaten an die externe Steuereinheit liefern. Insbesondere, jedoch nicht nur in diesen Fällen, kann es daher günstig sein, wenn die externe Steuereinheit Messdaten von wenigstens einem nicht aktivierten Sensor zwar empfängt, diese aber nicht verarbeitet. Die Aktivierung bzw. Nicht-Aktivierung des Sensors findet hier also in der externen Steuereinheit statt. Allen vorgenannten Aktivierungs- bzw. Nicht-Aktivierungs-Strategien ist gemeinsam, dass der Benutzer keine Daten der nicht aktivierten Sensoren zur Verfügung gestellt bekommt.

Bei einer bevorzugten Ausführungsform des Verfahrens ist der Behälter als ein Einweg-Mischbehälter oder ein Einweg-Bioreaktor mit jeweils wenigstens bereichsweise flexibler Wandung ausgelegt.

Bei einer besonders bevorzugten Ausführungsform des Verfahrens ist der Einweg-Bioreaktor als Beutel ausgelegt.

Bei einer weiteren bevorzugten Ausführungsform des Verfahrens ist der Behälter als ein Einweg-Mischbehälter oder ein Einweg-Bioreaktor mit jeweils wenigstens bereichsweise starrer Wandung ausgelegt, im Stand der Technik auch unter der Bezeichnung "Carboy" bekannt.

Allen vorgenannten Behältern ist gemeinsam, dass sie besonders kostengünstig aus Kunststoff gefertigt werden können.

Wie eingangs bereits erläutert, kann die Kommunikationsstrecke eines Sensors als Funkstrecke, elektrisch leitendes Kabel, als Glasfaserkabel oder auf andere Weise ausgebildet sein. Im Fall der Kabelankopplung mehrerer Sensoren ist es besonders vorteilhaft, wenn mehrere als elektrisch leitende Kabel und/oder mehrere als optische Glasfaserkabel ausgebildete Kommunikationsstrecken in einem standardisierten Sammelkabel zusammengefasst sind. Dies entspricht einer Weiterführung der Idee der undifferenzierten Massenproduktion, die hier auch auf die Kabelverbindung zwischen standardisiertem Sensorfeld und der externen Steuereinheit ausgeweitet wird. Auf diese Weise kann unabhängig von der konkreten Aktivierungs-Lizenzierung, d.h. unabhängig von der tatsächlichen Funktionalität des Einweg-Behälters zu seiner Ankopplung, stets das gleiche, wiederverwendbare Kabel genutzt werden.

Weitere Merkmale und Vorteile der Erfindung ergeben sich aus der nachfolgenden speziellen Beschreibung und der Zeichnung.

### Kurzbeschreibung der Zeichnung

Es zeigt:

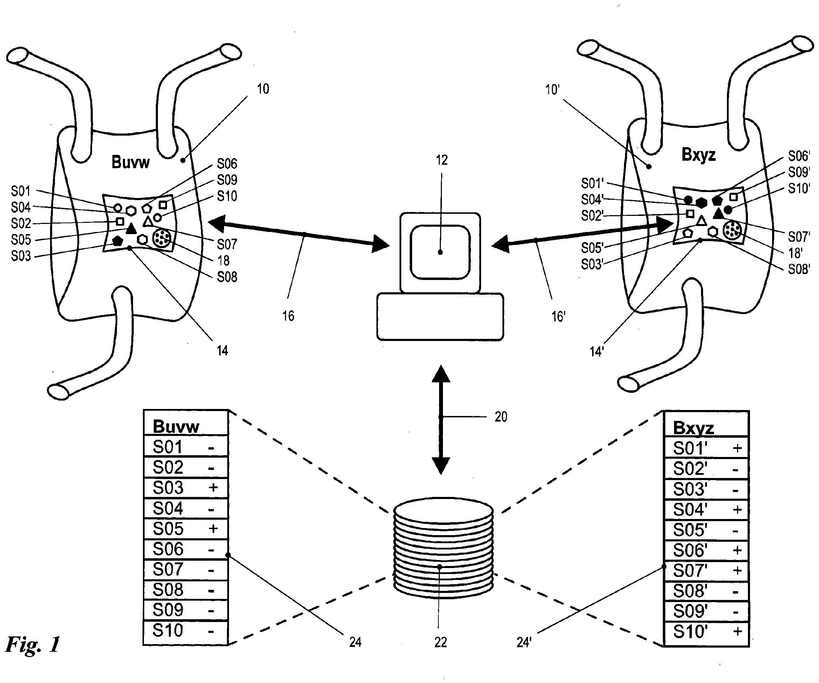

- Figur 1:: eine schematisierte Darstellung des erfindungsgemäßen Verfahrens.

### Ausführliche Beschreibung bevorzugter Ausführungsformen

Figur 1 zeigt in schematisierter Darstellung die grundsätzliche Funktionsweise des erfindungsgemäßen Verfahrens.

Dargestellt sind zwei identische Behälter mit flexibler Wandung, beispielsweise Einweg-Bioreaktorbeutel oder Einweg-Mischbehälter 10, 10'. Der in Figur 1 links dargestellte Behälter 10 trägt die als "Buvw" symbolisierte individuelle Kennung, der in Figur 1 rechts dargestellte Behälter 10' trägt die als "Bxyz" symbolisierte individuelle Kennung. Diese Kennung kann in Klarschrift, als Barcode, als QR-Code, als Transponder, als RFID-Chip oder auf ähnliche Weise realisiert sein. Wesentlich ist, dass die Kennung von einer externen Steuereinheit 12 gelesen werden kann, sodass die Behälter 10, 10' individuell erkannt werden können. Der Fachmann wird verstehen, dass es im Kontext der vorliegenden Erfindung nicht auf die individuelle Erkennung der Behälter als solche ankommt, sondern vielmehr auf die Erkennung der nachfolgend zu beschreibenden Sensoren, die herstellerseitig fest mit der Wandung der Beutel 10, 10' verbunden sind.

Beide Behälter 10, 10' tragen ein identisch ausgebildetes Sensorfeld 14, 14', welches eine fluiddicht mit der Wandung des jeweiligen Behälters 10, 10' verbundene Trägerplatte sowie fest mit der Trägerplatte verbundene Sensoren S01-S10 bzw. S01'-S10' umfasst. Die Sensoren S01-S10, S01'-S10' sind, wie durch die Kommunikationspfeile 16, 16' angedeutet, datenübertragend mit der externen Steuereinheit 12 verbunden. Diese datenübertragende Verbindung kann individuell von jedem Sensor S01-S10, S01'-S10' zur externen Steuereinheit 12 oder über eine mehrere Sensor-Ein/Ausgänge zusammenfassende Schnittstelle 18, 18' erfolgen. Selbstverständlich sind auch Kombinationen möglich, sodass bspw. einige der Sensoren S01-S10, S01'-S10' über Glasfasern oder elektrische Leiter kabelgebunden und andere der Sensoren S01-S10, S01'-S10' drahtlos mit der externen Steuereinheit 12 verbunden sind.

Zudem stehen die Sensoren S01-S10, S01'-S10' mit ihren nicht gesondert dargestellten Sensorköpfen mit dem Inneren des jeweiligen Behälters 10, 10' in Kontakt, sodass mit ihnen Zustandsparameter eines im Behälter 10, 10' enthaltenen Fluids messbar sind. Trotz der hardwareseitig identischen Ausbildung der Behälter 10, 10' und ihrer Sensorfelder 14, 14' unterscheiden sie sich bei der gezeigten Ausführungsform in ihrer Funktionalität erheblich. Die externe Steuereinheit 12 hat nämlich, wie durch den Kommunikationspfeil 20 angedeutet, Zugriff auf eine Datenbank 22, in welcher beutelindividuelle Aktivierungsdatensätze 24, 24' für die Sensoren S01-S10, S01'-S10' hinterlegt sind. Bei der dargestellten Ausführungsform sind für den Behälter Buvw (10), der beispielsweise als Beutel ausgelegt ist, die Sensoren S03 und S05 als aktivierbar hinterlegt, wohingegen die übrigen Sensoren, S01, S02, S04 und S06-S10 als nicht aktivierbar hinterlegt sind. In Figur 1 ist dies durch "+"- bzw. "-"-Zeichen im Aktivierungs-Datensatz 24 sowie durch volle oder leere Darstellung der Sensoren im Sensorfeld 14 angedeutet. Für den Behälter Bxyz 10' hingegen, der beispielsweise als Beutel ausgelegt ist, sind die Sensoren S01', S04', S06', S07' und S10' als aktivierbar hinterlegt, wohingegen die Sensoren S02', S03', S05', S08' und S09' als nicht aktivierbar hinterlegt sind. Die Darstellung im Aktivierungs-Datensatz 24' und im Sensorfeld 14' ist entsprechend.

Zur Durchführung von Messungen mit den Sensorfeldern 14, 14' greift die externe Steuereinheit zunächst auf die Aktivierungs-Datensätze 24, 24' zu und aktiviert dann für jeden Behälter 10, 10' lediglich diejenigen Sensoren, die dort als aktivierbar hinterlegt sind. Von den übrigen, als nicht aktivierbar hinterlegten Sensoren bekommt der Benutzer keinerlei Messdaten zur Verfügung gestellt. Dabei ist unerheblich, an welcher Stelle die Sensor-Blockade erfolgt. Wie im allgemeinen Teil der Beschreibung bereits diskutiert, kann dies auf Ebene des Sensorkopfs, auf Ebene der Kommunikationsstrecke zur externen Steuereinheit oder in der externen Steuereinheit selbst erfolgen.

Der Fachmann wird verstehen, dass dieses Verfahren ein breites Spektrum von Lizenzmanagement-Strategien erlaubt. Während der Hersteller den Vorteil genießt, lediglich eine Sorte identischer Behälter ohne hardwareseitige Individualkonfektionierung herstellen zu müssen, genießt der Benutzer den Vorteil über zu- und abbuchbare Lizenzen lediglich für diejenigen Funktionalitäten zahlen zu müssen, die er tatsächlich für seinen individuellen Prozess benötigt. Die durch die identische Massenproduktion im Gegensatz zur Individualkonfektion erzielten Kostenvorteile führen zu Einsparungen sowohl auf Hersteller- als auch auf Benutzerseite, insbesondere dann, wenn die Behälter als Einweg-Mischbehälter oder Einweg-Bioreaktor ausgelegt sind.

## Patentansprüche

1. Verfahren zum Messen einer Mehrzahl von Zustandsparametern eines in einem Behälter (10, 10') enthaltenen Fluids,
wobei an der Wandung des zum Einmalgebrauch konfigurierten Behälters (10, 10') eine Sensorträgerplatte fluiddicht festgelegt ist, welche eine Mehrzahl von Sensoren (S01-S10, S01'-S10') trägt, die jeweils einen mit dem Innenraum des Behälters (10, 10') in Wirkkontakt stehenden Sensorkopf und eine mit einer externen Steuereinheit (12) in datenaustauschender Verbindung stehende Kommunikationsstrecke aufweisen,
und wobei die externe Steuereinheit (12) mittels der Sensorköpfe aktiver Sensoren (S03, S05; S01', S04', S06', S07', S10') erzeugte Messdaten über deren Kommunikationsstrecke empfängt und verarbeitet,
**dadurch gekennzeichnet,**
**dass** jedem Sensor (S01-S10, S01'-S10') ein Aktivierungs-Datensatz (24, 24') zugeordnet und für die externe Steuereinheit (12) zugänglich hinterlegt ist und dass die externe Steuereinheit (12) zunächst auf die Aktivierungs-Datensätze (24, 24') zugreift, die Sensoren (S01-S10, S01-S10') dann nach vorgegebenen Regeln auf Basis ihrer jeweiligen, zugeordneten Aktivierungs-Datensätze (24, 24') als aktivierbare oder nicht-aktivierbare Sensoren einstuft und im Folgenden ausschließlich die als aktivierbare Sensoren eingestuften Sensoren (S03, S05; S01', S04', S06', S07', S10') aktiviert.

2. Verfahren nach Anspruch 1,
**dadurch gekennzeichnet,**
**dass** wenigstens ein nicht aktivierter Sensor (S01, S02, S04, S06-S10; S02', S03', S05', S08', S09') keine Messdaten erfasst.

3. Verfahren nach einem der vorangehenden Ansprüche,
**dadurch gekennzeichnet,**
**dass** wenigstens ein nicht aktivierter Sensor (S01, S02, S04, S06-S10; S02', S03', S05', S08', S09') Messdaten erfasst und die externe Steuereinheit (12) die Messdaten nicht empfängt.

4. Verfahren nach einem der vorangehenden Ansprüche,
**dadurch gekennzeichnet,**
**dass** die externe Steuereinheit (12) Messdaten von wenigstens einem nicht aktivierten Sensor (S01, S02, S04, S06-S10; S02', S03', S05', S08', S09') empfängt und diese nicht verarbeitet.

5. Verfahren nach einem der vorangehenden Ansprüche,
**dadurch gekennzeichnet,**
**dass** die Kommunikationsstrecke wenigstens eines Sensors (S01-S10, S01',S10') als Funkstrecke ausgebildet ist.

6. Verfahren nach einem der vorangehenden Ansprüche,
**dadurch gekennzeichnet,**
**dass** die Kommunikationsstrecke wenigstens eines Sensors (S01-S10, S01'-S10') als elektrisch leitendes Kabel ausgebildet ist.

7. Verfahren nach einem der vorangehenden Ansprüche,
**dadurch gekennzeichnet,**
**dass** die Kommunikationsstrecke wenigstens eines Sensors (S01-S10, S01-S10') als optisches Glasfaserkabel ausgebildet ist.

8. Verfahren nach den Ansprüchen 6 bis 7,
**dadurch gekennzeichnet,**
**dass** mehrere als elektrisch leitende Kabel und/oder mehrere als optische Glasfaserkabel ausgebildete Kommunikationsstrecken in einem standardisierten Sammelkabel zusammengefasst sind.

9. Verfahren nach einem der vorangehenden Ansprüche,
**dadurch gekennzeichnet,**
**dass** der Behälter (10, 10') als ein Einweg-Mischbehälter oder ein Einweg-Bioreaktor mit wenigstens bereichsweise flexibler oder starrer Wandung ausgelegt ist.

## Claims

1. A method for measuring a plurality of state parameters of a fluid contained in a container (10, 10'),
wherein a sensor carrier plate is fastened in a fluid-tight manner to the wall of the container (10, 10'), the container being configured for single use, which sensor carrier plate bears a plurality of sensors (S01-S10, S01'-S10'), each of which has a sensor head in effective contact with the interior of the container (10, 10') and a communication path having a data-exchanging connection path to an external control unit (12),
and wherein the external control unit (12) receives measured data produced by means of the sensor heads of active sensors (S03, S05; S01', S04', S06', S07', S10') via the communication path of said sensors and processes said measured data,
**characterized in that**
each sensor (S01-S10, S01'-S10') is assigned an activation data set (24, 24'), which is stored to be accessible to the external control unit (12),
and that the external control unit (12) first accesses the activation data sets (24, 24'), then classifies the sensors (S01-S10, S01'-S10') as activatable or non-activatable sensors in accordance with specified rules on the basis of the activation data sets (24, 24') assigned to the sensors, and thereafter activates only the sensors (S03, S05; S01', S04', S06', S07', S10') classified as activatable sensors.

2. The method according to claim 1,
**characterized in that**
at least one non-activated sensor (S01, S02, S04, S06-S10; S02', S03', S05', S08', S09') does not acquire measured data.

3. The method according to any of the preceding claims,
**characterized in that**
at least one non-activated sensor (S01, S02, S04, S06-S10; S02', S03', S05', S08', S09') acquires measured data and the external control unit (12) does not receive the measured data.

4. The method according to any of the preceding claims,
**characterized in that**
the external control unit (12) receives measured data from at least one non-activated sensor (S01, S02, S04, S06-S10; S02', S03', S05', S08', S09') and does not process the data.

5. The method according to any of the preceding claims,
**characterized in that**
the communication path of at least one sensor (S01-S10, S01',S10') is a radio path.

6. The method according to any of the preceding claims,
**characterized in that**
the communication path of at least one sensor (S01-S10, S01',S10') is an electrically conductive cable.

7. The method according to any of the preceding claims,
**characterized in that**
the communication path of at least one sensor (S01-S10, S01',S10') is an optical glass fiber cable.

8. The method according to claims 6 to 7,
**characterized in that**
multiple communication paths embodied as electrically-conductive cables and/or embodied as optical glass fiber cables are combined in a standardized composite cable.

9. The method according to any of the preceding claims,
**characterized in that**
the container (10, 10') is a single use mixing container or a one-time use bioreactor having a flexible or rigid wall in at least some areas.

## Revendications

1. Procédé permettant de mesurer une pluralité de paramètres d'état d'un fluide contenu dans un récipient (10, 10'),
une plaque de support de capteur étanche aux fluides étant fixée sur la paroi du récipient (10, 10') configuré à usage unique, ladite plaque de support de capteur étant pourvue d'une pluralité de capteurs (S01-S10, S01'-S10') qui présentent respectivement une tête de détection en contact fonctionnel avec l'intérieur du récipient (10, 10') et une voie de communication en contact de façon à échanger des données avec une unité de commande externe (12),
et l'unité de commande externe (12) recevant via la voie de communication des données de mesure générées au moyen des têtes de détection de capteurs actifs (S03, S05 ; S01', S04', S06', S07', S10') et traitant celles-ci,
**caractérisé en ce qu'**un
jeu de données d'activation (24, 24') est associé à chaque capteur (S01-S10, S01'-S10') et est stocké de manière accessible pour l'unité de commande externe (12),
et **en ce que** l'unité de commande externe (12) accède d'abord aux jeux de données d'activation (24, 24'), classe ensuite les capteurs (S01-S10, S01'-S10'), en suivant des règles prédéfinies sur la base des différents jeux de données d'activation (24, 24') associés à chaque capteur, en tant que capteurs pouvant être activés ou capteurs ne pouvant pas être activés, puis active uniquement les capteurs pouvant être activés (S03, S05 ; S01', S04', S06', S07', S10').

2. Procédé selon la revendication 1,
**caractérisé en ce qu'**au
moins un capteur non activé (S01, S02, S04, S06-S10 ; S02', S03', S05', S08', S09') n'enregistre pas de données de mesure.

3. Procédé selon l'une quelconque des revendications précédentes,
**caractérisé en ce qu'**au
moins un capteur non activé (S01, S02, S04, S06-S10 ; S02', S03', S05', S08', S09') enregistre des données de mesure, et l'unité de commande externe (12) ne reçoit pas les données de mesure.

4. Procédé selon l'une quelconque des revendications précédentes,
**caractérisé en ce que**
l'unité de commande externe (12) reçoit des données de mesure d'au moins un capteur non activé (S01, S02, S04, S06-S10 ; S02', S03', S05', S08', S09') et ne traite pas lesdites données.

5. Procédé selon l'une quelconque des revendications précédentes,
**caractérisé en ce que**
la voie de communication d'au moins un capteur (S01-S10, S01'-S10') est réalisée sous forme de voie radioélectrique.

6. Procédé selon l'une quelconque des revendications précédentes,
**caractérisé en ce que**
la voie de communication d'au moins un capteur (S01-S10, S01'-S10') est réalisée sous forme de câble électriquement conducteur.

7. Procédé selon l'une quelconque des revendications précédentes,
**caractérisé en ce que**
la voie de communication d'au moins un capteur (S01-S10, S01-S10') est réalisée sous forme de câble optique en fibres de verre.

8. Procédé selon les revendications 6 à 7,
**caractérisé en ce que**
plusieurs voies de communication réalisées sous forme de câbles électriquement conducteurs et/ou plusieurs voies de communication réalisées sous forme de câbles optiques en fibres de verre sont regroupées dans un câble collecteur standardisé.

9. Procédé selon l'une quelconque des revendications précédentes,
**caractérisé en ce que**
le récipient (10, 10') est conçu comme un récipient mélangeur à usage unique ou un bioréacteur à usage unique, ayant une paroi flexible ou rigide au moins dans certaines zones.
